# EUROPEAN PATENT SPECIFICATION

(11) **EP 1 260 218 B1**
(45) Date of publication and mention of the grant of the patent: **20.07.2011**
(21) Application number: 02253341.8
(22) Date of filing: 14.05.2002
(51) Int. Cl.: A61K 9/28

(54) **Dip coating compositions containing starch or dextrin**
Tauchbeschichtungszusammensetzungen enthaltend Stärke oder Dextrin
Composition d'enrobage par trempage comprenant de l'amidon ou de la dextrine

(30) Priority: 15.05.2001 US 291127 P; 28.09.2001 US 325726 P; 12.04.2002 US 122999; 15.04.2002 US 122531
(43) Date of publication of application: 27.11.2002
(73) Proprietor: McNeil-PPC, Inc., Skillman, NJ 08558-9418 (US)
(72) Inventor: Gulian, Cynthia, Lansdale, PA 19446 (US); Gowan, Walter G., Jr., Woodstock, GA 30189 (US); Szymczak, Christopher, Marlton, NJ 08053 (US); Papalini, Michele, Philadelphia, PA 19106 (US); Chen, Jen-Chi, Morrisville, PA 19067 (US); Bunick, Frank J., Randolph, NJ 07869 (US)
(74) Representative: Mercer, Christopher Paul

(56) References cited:
- EP-A- 0 684 301
- EP-A- 0 717 992
- WO-A-01/03677
- WO-A-01/26634
- WO-A-01/91721
- WO-A-95/03063
- WO-A-98/30341
- FR-A- 2 783 832
- US-A- 3 751 277
- US-A- 4 828 841

## Description

### FIELD OF THE INVENTION

This invention relates to novel pharmaceutical dosage forms comprising, water soluble, gelatin-free compositions for dip coating substrates, such as tablets and capsules, and methods for producing such dosage forms. This invention further relates to a method for increasing the weight gain of a water soluble, gelatin-free, film forming coating on a dip-coated tablet or caplet.

### BACKGROUND OF THE INVENTION

During most of this century, hard gelatin capsules were a popular dosage form for prescription and over-the-counter (OTC) drugs. The ability to combine capsule halves having different colors provided manufacturers with a unique means of distinguishing various pharmaceutical products. Many patients preferred capsules over tablets, perceiving them as being easier to swallow. This consumer preference prompted pharmaceutical manufacturers to market certain products in capsule form even when they were also available in tablet form.

Generally, empty hard gelatin capsules are manufactured using automated equipment. This equipment employs rows of stainless steel pins, mounted on bars or plates, which are dipped into a gelatin solution maintained at a uniform temperature and fluidity. The pins are then withdrawn from the gelatin solution, rotated, and then inserted into drying kilns through which a strong blast of filtered air with controlled humidity is forced. A crude capsule half is thus formed over each pin during drying. Each capsule half is then stripped, trimmed to uniform length, filled and joined to an appropriate mating half.

An alternative to capsule products are caplets, which are solid, oblong tablets that are often coated with various polymers such as cellulose ethers to improve their aesthetics, stability, and swallowability. Typically, such polymers are applied to the tablets either from solution in organic solvents, or from aqueous dispersion via spraying. However, such spray-coated tablets lack the shiny surface and elegance of the hard gelatin capsules. Additionally, it is not commercially feasible to spray-coat a tablet with a different color coating on each end.

Another alternative to capsule products are "gelcaps," which are elegant, shiny, consumer-preferred dosage forms that are prepared by dipping each half of an elongated tablet in two different colors of gelatin solution. See United States Patent Nos.: 4,820,524; 5,538,125; 5,685,589; 5,770,225; 5,198,227; and 5,296,233, which are all incorporated by reference herein. A similar dosage form, commercially available as a "geltab," is prepared by dipping each half of a round, convex tablet into different colors of gelatin solution, as described in United States Patent Nos. 5,228,916, US5,436,026 and US5,679,406, which are all incorporated by reference herein. As used herein, such "gelcaps" and "geltabs" shall be included within the broader term, "tablets."

However, the use of gelatin as a pharmaceutical coating material presents certain disadvantages and limitations, including the potential for decreased dissolution rate after extended storage due to cross-linking of the gelatin, potential for microbial contamination of the gelatin solution during processing, and long processing times due to extensive drying requirements. Further, the energy-related costs associated with gelatin coatings tend to be high since the gelatin material is typically applied to the substrates at an elevated temperature of at least about 40°C in order to maintain fluidity of the gelatin, while the substrates are maintained at about 50°C in order to minimize microbial growth.

Various attempts have been made to produce gelatin-free hard shell capsules. For example, WO 00/18835 discloses the combination of starch ethers or oxidized starch and hydrocolloids for use in preparing hard capsule shells via conventional dip molding processing. See also U.S. Pat. No. 4,001211 (capsules prepared via pin dip coating with thermogelled methylcellulose ether compositions ). However, due to potential tampering concerns, hard gelatin capsules are no longer a preferred delivery system for consumer (over-the-counter) pharmaceuticals, dietary supplements, or other such products. Additionally, the properties of an ideal composition into which steel pins are to be dipped then dried to form hard capsule shells thereon are not necessarily the same as those for dipping tablets to form a coating thereon. For example, relevant physical properties such as viscosity, weight-gain, film thickness, tensile strength, elasticity, and moisture content will differ between compositions for hard capsule formation and for coating tablets. See e.g., U.S. Pat. No. 1,787,777 (Optimal temperatures of the substrate and coating solution, residence times in the solution, and drying conditions differ.)

WO 9830341 discloses a dry film coating composition for forming a coating suspension for film coating nutritional supplements, pharmaceutical tablets, and the like, comprising a dextrin and a detackifier.

US 3751277 discloses a composition for coating tablets and other individual dosage forms comprising a sugar, hydrolyzed cereal solids, starch, a solid polyethylene glycol and a liquid polyhydroxy organic compound is disclosed. The composition can be employed to coat tablets by applying aqueous solutions thereof to the tablets and drying the solvent.

One disadvantage associated with dipping tablets or capsules into a non-gelatin coating system is that the resulting coatings often lack adequate tensile strength, plasticity, hardness, and thickness. Moreover, the inclusion of plasticizers into such non-gelatin coating systems often results in tablets having soft, tacky coatings without a hardness sufficient to maintain their shape or smoothness during handling. In addition, many non-gelatin compositions do not adhere to the tablet substrate in an amount sufficient to uniformly cover the tablet after a single dipping. Further, many non-gelatin compositions lack the sufficient rheological properties necessary to maintain uniform color dispersion throughout the dipping and drying process. Although attempts have been made to improve the rheological properties of these compositions by, for example, increasing their solids content in order to increase viscosity. However, such compositions often disadvantageously resulted in undesirable coating aesthetics such as surface roughness, decreased gloss, and non-uniform coating thickness.

It is desirable to find a dip coating material, which not only produces a similar elegant, shiny, high gloss, consumer-preferred dosage form similar to that of gelatin-coated forms, but which is absent the limitations of gelatin, particularly those noted above.

### SUMMARY OF THE INVENTION

The present invention provides for a pharmaceutical dosage form comprising a core and a dip-coated coating layer; said coating layer substantially covering said core, characterised in that said coating layer is comprised of, based upon the total dry weight of the coating layer:
a) from 40 percent to 60 percent of a film former selected from the group consisting of a waxy maize starch, tapioca dextrin, a derivative of a waxy maize starch, a derivative of tapioca dextrin and mixtures thereof;
b) from 5 percent to 25 percent thickener selected from the group consisting of sucrose, dextrose, fructose and mixtures thereof; and
c) from 15 percent to 30 percent plasticizer,
wherein the coating layer possesses a surface gloss of at least 150.

The present invention additionally provides for a pharmaceutical dosage form comprising a core and a dip-coated coating layer; said coating layer substantially covering said core, characterised in that said coating layer is comprised of, based upon the total dry weight of the coating layer:
a) from 25 percent to 80 percent hydroxypropyl starch film former;
b) from 0.10 percent to 33 percent thickener selected from the group consisting of kappa carrageenan, iota carrageenan, maltodextrin, gellan gum, agar, gelling starch and derivatives and mixtures thereof; and
c) 11 percent to 60 percent of a plasticizer,
wherein the coating layer possesses a surface gloss of at least 150 and has a thickness of 40 microns to 400 microns.

The present invention additionally provides for a simulated capsule-like medicament comprising:
a. a core having a first and a second end,
b. a first coating layer having a first color provided on said first end of said core;
c. a second coating layer having a second color on said second end of said core, said first color is different than said second color;
characterised in that at least one of said first coating layer and second coating layer is the coating layer as described herein.

The present invention additionally provides for a method of making coated tablets from an aqueous dispersion comprising the coating layer as described herein comprising dip coating tablets in the aqueous dispersion.

We have found that when a dosage form is coated as described herein, the result is an elegant, shiny, high gloss, consumer-preferred dosage form similar to that of a gelatin-coated form, but which lacks the limitations associated with gelatin, particularly those noted above. We have also found that when such a composition is used in dip coating, it does not inhibit the dissolution of the active coated therewith. Further, we have found that the color uniformity of dosage forms coated with such compositions is improved upon the addition of a weight gain enhancer thereto.

### DETAILED DESCRIPTION OF THE INVENTION

As used herein, "capsules" refer to hard shell compartments that enclose a dosable ingredient. "Tablets," as used herein, refer to compressed or molded solid dosage forms of any shape or size. "Caplets," as used herein, refer to solid, oblong-shaped tablets. "Gelcaps" refer to solid caplets having a glossy gelatinous coating, and "geltabs" refer to solid tablets having flat sides, convex opposing faces, and a glossy gelatinous coating. "Hardness" as used herein in connection with films or coatings indicates the resistance of the film/coating to deformation upon impact. "Water soluble," as used herein in connection with non-polymeric materials, shall mean from sparingly soluble to very soluble, i.e., not more than 100 parts water required to dissolve 1 part of the non-polymeric, water soluble solute. See Remington, "The Science and Practice of Pharmacy," pages 208 - 209 (2000). "Water soluble," as used herein in connection with polymeric materials, shall mean that the polymer swells in water and can be dispersed at the molecular level to form a homogeneous dispersion or colloidal "solution." "Surface gloss" as used herein, shall refer to amount of light reflectance as measured at a 60 degree incident angle using the method set forth in Example 7 herein.

Dimethicone is a well known pharmaceutical material consisting of linear siloxane polymers containing repeating units of the formula {-(CH₂)₂SiO}ₙ stabilized with trimethylsiloxy end blocking units of the formula [(CH₃)₃SiO-]. Simethicone is the mixture of dimethicone and silicon dioxide. For the purposes of this invention, the two materials may be used interchangably.

The composition for use in the present invention may comprise, consist of, and/or consist essentially of a film former such as hydroxypropyl starch; a thickener selected from kappa or iota carrageenan, maltodextrin, gellan gum, agar, gelling starches, and derivatives and mixtures thereof; and a plasticizer. As used herein, "substantially gelatin-free" shall mean less than 1 percent, e.g. less than 0.5 percent, of gelatin in the composition, and "substantially free of thickeners" shall mean less than 1 percent, e.g. less than 0.01 percent, of thickeners in the composition.

Suitable film formers for use in film forming composition of the present invention are hydroxypropyl starch, waxy maize starch, tapioca dextrin, a derivative of waxy maize starch, a derivative of tapioca dextrin and mixtures thereof.

As used herein, HPMC is hydroxypropylmethylcellulose compound which may be "HPMC 2910", which is a cellulose ether having a degree of substitution of about 1.9 and a hydroxypropyl molar substitution of 0.23, and containing, based upon the total weight of the compound, from 29% to 30% methoxyl and from 7% to 12% hydroxylpropyl groups. HPMC 2910 is commercially available from the Dow Chemical Company under the tradename, "Methocel E." "Methocel E5, " which is one grade of HPMC-2910 suitable for use in the present invention, has a viscosity of 4 to 6 cps (4 to 6 millipascal-seconds) at 20 °C in a 2% aqueous solution as determined by a Ubbelohde viscometer. Similarly, "Methocel E6 ," which is another grade of HPMC-2910 suitable for use in the present invention, has a viscosity of 5 to 7 cps ( 5 to 7 millipascal-seconds) at 20 °C in a 2% aqueous solution as determined by a Ubbelohde viscometer. "Methocel E15," which is another grade of HPMC-2910 suitable for use in the present invention, has a viscosity of about 15000 cps (15 millipascal-seconds) at 20 °C in a 2% aqueous solution as determined by a Ubbelohde viscometer. As used herein, "degree of substitution" shall mean the average number of substituent groups attached to an anhydroglucose ring, and "hydroxypropyl molar substitution" shall mean the number of moles of hydroxypropyl per mole anhydroglucose.

As used herein, "modified starches" include starches that have been modified via crosslinking and/or other chemical modification for improved stability or optimized performance, or physical modification for improved solubility properties or optimized performance. Examples of chemically-modified starches are well known in the art and typically include those starches that have been chemically treated to cause replacement of some of its hydroxyl groups with either ester or ether groups. Crosslinking, as used herein, may occur in modified starches when two hydroxyl groups on neighboring starch molecules are chemically linked. As used herein, "pre-gelatinized starches" or "instantized starches" refers to physically modified starches that have been pre-wetted, then dried to enhance their cold-water solubility. Suitable modified starches are commercially available from several suppliers such as, for example, A.E. Staley Manufacturing Company, and National Starch & Chemical Company.

A suitable film forming modified starch includes the pre-gelatinized waxy maize derivative starches that are commercially available from National Starch & Chemical Company under the tradenames, "Purity Gum" and "FilmSet", and derivatives, copolymers, and mixtures thereof. Such waxy maize starches typically contain, based upon the total weight of the starch, from 0 percent to 18 percent of amylose and from 100 percent to 88 percent of amylopectin.

Another suitable film forming modified starch includes the hydroxypropylated starches, in which some of the hydroxyl groups of the starch have been etherified with hydroxypropyl groups, usually via treatment with propylene oxide. One example of a suitable hydroxypropyl starch that possesses film-forming properties is available from Grain Processing Company under the tradename, "Pure-Cote B790".

Suitable film forming tapioca dextrins include those available from National Starch & Chemical Company under the tradename, "Crystal Gum" or "K-4484," and derivatives thereof such as modified food starch derived from tapioca, which is available from National Starch and Chemical under the tradename, "Purity Gum 40," and copolymers and mixtures thereof.

Suitable thickeners are agar, kappa carrageenan, iota carrageenan, gellan gum, maltodextrin, and gelling starches such as acid hydrolyzed starches and derivatives and mixtures thereof. Additional suitable thickeners include sucrose, dextrose, fructose, maltodextrin, and combinations thereof.

"Acid-hydrolyzed starch," as used herein, is one type of modified starch that results from treating a starch suspension with dilute acid at a temperature below the gelatinization point of the starch. During the acid hydrolysis, the granular form of the starch is maintained in the starch suspension, and the hydrolysis reaction is ended by neutralization, filtration and drying once the desired degree of hydrolysis is reached. As a result, the average molecular size of the starch polymers is reduced. Acid-hydrolyzed starches (also known as "thin boiling starches") tend to have a much lower hot viscosity than the same native starch as well as a strong tendency to gel when cooled.

"Gelling starches," as used herein, include those starches that, when combined with water and heated to a temperature sufficient to form a solution, thereafter form a gel upon cooling to a temperature below the gelation point of the starch. Examples of gelling starches include, but are not limited to, acid hydrolyzed starches such as that available from Grain Processing Corporation under the tradename, "Pure-Set B950"; hydroxypropyl distarch phosphate such as that available from Grain Processing Corporation under the tradename, "Pure-Gel B990", and mixtures thereof.

Any plasticizer known in the pharmaceutical art is suitable for use in the present invention, and may include, but not be limited to polyethylene glycol; glycerin; sugar alcohols; triethyl citrate; tribuyl citrate; dibutyl sebecate; vegetable oils such as castor oil; surfactants such as polysorbates, sodium lauryl sulfates, and dioctyl-sodium sulfosuccinates; propylene glycol; mono acetate of glycerol; diacetate of glycerol; triacetate of glycerol; natural gums and mixtures thereof. Suitable sugar-alcohols include sorbitol, mannitol, xylitol, maltitol, erythritol, lactitol, and mixtures thereof. In solutions containing a cellulose ether film former, an optional plasticizer may be present in an amount, based upon the total weight of the solution, from about 0 percent to about 40 percent.

In one embodiment, the film forming composition for dip coating substrates may be substantially free of gelatin, i.e., e.g. contains less than about 1%, or less than about 0.01% of gelatin.

In another embodiment, the film forming composition for dip coating substrates may be substantially free of bovine derived materials, i.e., e.g. contains less than about 1%, or less than about 0.01% of bovine derived materials.

In embodiments wherein a cellulose ether film former is used in the composition, the film forming composition for dip coating substrates may be substantially free of hydrocolloids, i.e., e.g. contains less than about 1%, or less than about 0.01% of hydrocolloids.

In one embodiment, the film forming composition for dip coating substrates contains, based upon the total dry solids weight of the composition, from 40 percent to 60 percent, e.g. from 50 percent to 55 percent, of a modified starch, e.g. a waxy maize starch, a tapioca dextrin, and/or mixtures and derivatives thereof; from 15 percent to 30 percent, e.g., from 20 percent to 25 percent of a plasticizer, e.g., glycerin, polyethylene glycol, propylene glycol, castor oil, and mixtures thereof; and from 5 percent to 25 percent, e.g., from 10 percent to 20 percent, of a thickener, e.g., sucrose, dextrose, fructose, maltodextrin, polydextrose, and mixtures thereof.

In another embodiment, the film forming composition for dip coating substrates contains, based upon the total dry solids weight of the composition, from 25 percent to 80 percent, e.g. from 50 to 75 percent, of a film former such as a chemically modified starch, e.g. hydroxypropyl starch; from 0.10 percent to 33 percent, e.g. from 0.15 percent to 1 percent, or from 10 percent to 25 percent of a thickening agent; and from 11 percent to 60 percent, e.g. from 20 percent to 40 percent of a plasticizer.

In one embodiment wherein the film former is a chemically modified starch, the thickener may be selected from the group consisting of kappa or iota carrageenan, maltodextrin, gellan gum, agar, gelling starch and derivatives and mixtures thereof.

In one embodiment wherein the film former is a chemically modified starch, the plasticizer may be selected from the group consisting of glycerin, propylene glycol, polyethylene glycol, sugar alcohols and derivatives and mixtures thereof.

The film forming compositions for use in the present invention are typically in the form of a dispersion for ease of dip coating substrates therein. Such dispersions contain a solvent in an amount, based upon the total weight of the dispersion, from 30 percent to 97 percent, for example, from 80 percent to 92 percent or from 40 percent to 75 percent. Examples of suitable solvents include, but are not limited to water; alcohols such as methanol, ethanol, and isopropanol; organic solvents such as methylene chloride, acetone, and the like; and mixtures thereof. In one embodiment, the solvent is water. The resulting film forming dispersion typically possesses a solids level of, based upon the total weight of the film forming dispersion, from 3 percent to 70 percent, for example, from 8 percent to 20 percent or from 25 percent to 60 percent.

In another embodiment, the film forming composition for dip coating substrates contains, based upon the total wet weight of the dipping dispersion composition, from 20 percent to 35 percent, e.g. from 25 percent to 30 percent, of a film former such as waxy maize starch, tapioca dextrin, and/or derivatives and mixtures thereof; from 5 percent to 20 percent, e.g., from 10 percent to 15 percent of a plasticizer such as glycerin, polyethylene glycol, propylene glycol, castor oil, and mixtures thereof; and from 5 percent to 15 percent of a thickener selected from sucrose, fructose, dextrose, maltodextrin, polydextrose, and mixtures thereof.

In yet another embodiment, the film forming composition for dip coating substrates contains, based upon the total wet weight of the dipping dispersion composition, from 15 percent to 30 percent, e.g. from 20 to 25 percent, of a film former such as a chemically modified starch, e.g. hydroxypropyl starch; from 0.05 percent to 10 percent, e.g. from 0.15 percent to 7 percent of a thickening agent; and from 5 percent to 20 percent, e.g. from 8 percent to 12 percent of a plasticizer.

.

Optionally, the composition for dipping may further comprise other ingredients such as, based upon the total weight of the dipping solution, from 0 percent to 2 percent preservatives such as methylparaben and propylparaben, from 0 percent to 14 percent opacifying agents such as titanium dioxide, and/or from 0 percent to 14 percent colorants. See Remington's Practice of Pharmacy, Martin & Cook, 17th ed., pp. 1625 - 30.

Any coloring agent suitable for use in pharmaceutical applications may be used in the present invention and may include, but not be limited to azo dyes, quinopthalone dyes, triphenylmethane dyes, xanthene dyes, indigoid dyes, iron oxides, iron hydroxides, titanium dioxide, natural dyes, and mixtures thereof. More specifically, suitable colorants include, but are not limited to patent blue V, acid brilliant green BS, red 2G, azorubine, ponceau 4R, amaranth, D&C red 33, D+C red 22, D+C red 26, D+C red 28, D+C yellow 10, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, titanium dioxide, riboflavin, carotenes, antyhocyanines, turmeric, cochineal extract, clorophyllin, canthaxanthin, caramel, betanin, and mixtures thereof.

In one embodiment, each end of the tablet or capsule may be coated with dip coatings of different colors to provide a distinctive appearance for specialty products. See United States Patent No. 4,820,524.

In one embodiment, the pharmaceutical dosage form is comprised of a) a core containing an active ingredient; b) an optional first coating layer comprised of a subcoating that substantially covers the core; and c) a second coating layer on the surface of the first coating layer, the second coating layer comprised of the dip coating composition of the present invention. As used herein, "substantially covers" shall mean at least 95 percent of the surface area of the core is covered by the subcoating.

In an alternate embodiment, a first active ingredient may be contained in the first coating layer, and the core may contain a second active ingredient and/or an additional amount of the first active ingredient. In yet another embodiment, the active ingredient may be contained in the first coating layer, and the core may be substantially free, i.e., less than 1 percent, e.g. less than 0.1 percent, of active ingredient.

The use of subcoatings is well known in the art and disclosed in, for example, United States Patent Nos. 3,185,626. Any composition suitable for film-coating a tablet may be used as a subcoating according to the present invention. Examples of suitable subcoatings are disclosed in United States Patent Nos. 4,683,256, 4,543,370, 4,643,894, 4,828,841, 4,725,441, 4,802,924, 5,630,871, and 6,274,162. Additional suitable subcoatings include one or more of the following ingredients: cellulose ethers such as hydroxypropylmethylcellulose, hydroxypropylcellulose, and hydroxyethylcellulose; polycarbohydrates such as xanthan gum, starch, and maltodextrin; plasticizers including for example, glycerin, polyethylene glycol, propylene glycol, dibutyl sebecate, triethyl citrate, vegetable oils such as castor oil, surfactants such as polysorbate-80, sodium lauryl sulfate and dioctyl-sodium sulfosuccinate; polycarbohydrates, pigments, and opacifiers.

In one embodiment, the subcoating may be comprised of, based upon the total weight of the subcoating, from 2 percent to 8 percent, e.g. from 4 percent to 6 percent of a water-soluble cellulose ether and from 0.1 percent to 1 percent, castor oil, as disclosed in detail in United States Patent No. 5,658, 589. In another embodiment, the subcoating may be comprised of, based upon the total weight of the subcoating, from 20 percent to 50 percent, e.g., from 25 percent to 40 percent of HPMC; from 45 percent to 75 percent, e.g., from 50 percent to 70 percent of maltodextrin; and from 1 percent to 10 percent, e.g., from 5 percent to 10 percent of PEG 400.

The dried subcoating typically is present in an amount, based upon the dry weight of the core, from 0 percent to 5 percent. The dried dip coating layer typically is present in an amount, based upon the dry weight of the core and the optional subcoating, from 1.5 percent to 10 percent.

The average thickness of the dried dip coating layer typically is from 40 to 400 microns. However, one skilled in the art would readily appreciate without undue experimentation that the dip coating thickness may be varied in order to provide a smoother, easier to swallow, dosage form or to achieve a desired dissolution profile. Moreover, the thickness of dipped film coatings may vary at different locations on the substrate depending upon its shape. For example, the thickness of the coating at an edge or corner of a substrate may be as much as 50 percent to 70 percent less than the thickness of the coating at the center of a major face of the substrate. This difference can be minimized by, for example, use of a thicker subcoating, or use of dipping compositions that result in higher weight gains on the substrate.

In embodiments wherein a thicker dip coating is desired, we have found that an effective amount of a weight gain enhancer selected from the group consisting of simethicone, polysorbate 80 and mixtures thereof, may be added to a film forming composition comprised, consisting of, and/or consisting essentially of a film former and an optional thickener such as a hydrocolloid. The weight gain enhancer is used in an amount sufficient to increase the weight gain of the coating solution, e.g. by at least 10 percent, by at least 20%, or by at least 30 % on a substrate when dried. The percent weight gain increase is determined based upon the difference between the total weight of the coated substrate with the coating composition including the weight gain enhancer, and the total weight of an coated equivalent substrate, which has been coated under similar processing conditions with a coating composition that does not include an effective amount of weight gain enhancer.

A suitable film forming composition capable of achieving increased weight gain of dip coating on a substrate may contain, based upon the total dry weight of the film forming composition, from about 40 percent to about 99.9 percent, e.g. from about 95 percent to about 99.5 percent, or from about 40 percent to about 60 percent of a film former; from about 0 percent to about 60 percent , e.g. from about 0 percent to about 10 percent, or from about 0.5 percent to about 5 percent, or from about 10 percent to about 25 percent of a thickener; and from about 0.01 percent to about 0.25 percent, e.g. from about 0.03 percent to about 0.15 percent of a weight gain enhancer. When aesthetics of the final tablet are of particular concern, it is recommended to not use greater than about 0.25 percent of a weight gain enhancer. As shown above, the amount of thickener suitable for use in the composition will vary depending upon, for example, the particular thickener selected and the desired properties of the coating.

The film forming compositions of the present invention may be prepared by combining the film former, the thickener, and any optional ingredients such as plasticizers, preservatives, colorants, opacifiers, the weight gain enhancer, or other ingredients with the solventusing a high shear mixer until homogeneous under ambient conditions. In embodiments wherein a waxy maize starch derivative is used as a film former, the mixture may be heated to a temperature of 60 °C to 90 °C for faster dispersion of the ingredients. Alternatively, the film former and thickener may be preblended as dry powders, followed by addition of the resulting powder blend to the water and optional weight gain enhancer with high speed mixing. In order to remove substantially all of the bubbles from the resulting mixture, the pressure may then be decreased to about 5 inches Hg while reducing the mixing speed in order to avoid creating a vortex therein. Any other additional optional ingredients may then be added thereto at constant mixing.

It has surprisingly been found that substrates may be dipped into such solutions of the present invention using the same equipment and similar range of process conditions as used for the production of dip molded, gelatin-coated tablets. For example, both tablets and hard capsules may be coated using the aqueous dispersions of the present invention via known gelatin-dipping process parameters and equipment. Details of such equipment and processing conditions are known in the art and are disclosed at, for example, United States Patent No. 4,820,524. Advantageously, because the coating solutions of the present invention are fluid at room temperature and are less susceptible to microbial growth than gelatin compositions, the dip coating process may occur under ambient temperature and pressure conditions.

The tablets dip coated with the composition of the present invention may contain one or more active agents. The term "active agent" is used herein in a broad sense and may encompass any material that can be carried by or entrained in the system. For example, the active agent can be a pharmaceutical, nutraceutical, vitamin, dietary supplement, nutrient, herb, foodstuff, dyestuff, nutritional, mineral, supplement, or favoring agent or the like and combinations thereof.

The active agents useful herein can be selected from classes from those in the following therapeutic categories: ace-inhibitors; alkaloids; antacids; analgesics; anabolic agents; anti-anginal drugs; anti-allergy agents; anti-arrhythmia agents; antiasthmatics; antibiotics; anticholesterolemics; anticonvulsants; anticoagulants; antidepressants; antidiarrheal preparations; anti-emetics; antihistamines; antihypertensives; anti-infectives; anti-inflammatories; antilipid agents; antimanics; anti-migraine agents; antinauseants; antipsychotics; antistroke agents; antithyroid preparations; anabolic drugs; antiobesity agents; antiparasitics; antipsychotics; antipyretics; antispasmodics; antithrombotics; antitumor agents; antitussives; antiulcer agents; anti-uricemic agents; anxiolytic agents; appetite stimulants; appetite suppressants; beta-blocking agents; bronchodilators; cardiovascular agents; cerebral dilators; chelating agents; cholecystekinin antagonists; chemotherapeutic agents; cognition activators; contraceptives; coronary dilators; cough suppressants; decongestants; deodorants; dermatological agents; diabetes agents; diuretics; emollients; enzymes; erythropoietic drugs; expectorants; fertility agents; fungicides; gastrointestinal agents; growth regulators; hormone replacement agents; hyperglycemic agents; hypoglycemic agents; ion-exchange resins; laxatives; migraine treatments; mineral supplements; mucolytics, narcotics; neuroleptics; neuromuscular drugs; non-steroidal anti-inflammatories (NSAIDs); nutritional additives; peripheral vasodilators; polypeptides; prostaglandins; psychotropics; renin inhibitors; respiratory stimulants; sedatives; steroids; stimulants; sympatholytics; thyroid preparations; tranquilizers; uterine relaxants; vaginal preparations; vasoconstrictors; vasodilators; vertigo agents; vitamins; wound healing agents; and others.

Active agents that may be used in the invention include, but are not limited to: acetaminophen; acetic acid; acetylsalicylic acid, including its buffered forms; acrivastine; albuterol and its sulfate; alcohol; alkaline phosphatase; allantoin; aloe; aluminum acetate, carbonate, chlorohydrate and hydroxide; alprozolam; amino acids; aminobenzoic acid; amoxicillin; ampicillin; amsacrine; amsalog; anethole; ascorbic acid; aspartame; astemizole; atenolol; azatidine and its maleate; bacitracin; balsam peru; BCNU (carmustine); beclomethasone diproprionate; benzocaine; benzoic acid; benzophenones; benzoyl peroxide; benzquinamide and its hydrochloride; bethanechol; biotin; bisacodyl; bismuth subsalicylate; bornyl acetate; bromopheniramine and its maleate; buspirone; caffeine; calamine; calcium carbonate, casinate and hydroxide; camphor; captopril; cascara sagrada; castor oil; cefaclor; cefadroxil; cephalexin; centrizine and its hydrochloride; cetirizine; cetyl alcohol; cetylpyridinium chloride; chelated minerals; chloramphenicol; chlorcyclizine hydrochloride; chlorhexidine gluconate; chloroxylenol; chloropentostatin; chlorpheniramine and its maleates and tannates; chlorpromazine; cholestyramine resin; choline bitartrate; chondrogenic stimulating protein; cimetidine; cinnamedrine hydrochloride; citalopram; citric acid; clarithromycin; clemastine and its fumarate; clonidine; clorfibrate; cocoa butter; cod liver oil; codeine and its fumarate and phosphate; cortisone acetate; ciprofloxacin HCI; cyanocobalamin; cyclizine hydrochloride; cyproheptadine; danthron; dexbromopheniramine maleate; dextromethorphan and its hydrohalides; diazepam; dibucaine; dichloralphenazone; diclofen and its alkali metal sales; diclofenac sodium; digoxin; dihydroergotamine and its hydrogenates/mesylates; diltiazem; dimethicone; dioxybenzone; diphenhydramine and its citrate; diphenhydramine and its hydrochloride; divalproex and its alkali metal salts; docusate calcium, potassium, and sodium; doxycycline hydrate; doxylamine succinate; dronabinol; efaroxan; enalapril; enoxacin; ergotamine and its tartrate; erythromycin; estropipate; ethinyl estradiol; ephedrine; epinephrine bitartrate; erythropoietin; eucalyptol; famotidine; fenoprofen and its metal salts; ferrous fumarate, gluconate and sulfate; fexofenadine; fluoxetine; folic acid; fosphenytoin; 5-fluorouracil (5-FU); fluoxetine; flurbiprofen; furosemide; gabapentan; gentamicin; gemfibrozil; glipizide; glycerine; glyceryl stearate; granisetron; griseofulvin; growth hormone; guafenesin; hexylresorcinol; hydrochlorothiazide; hydrocodone and its tartrates; hydrocortisone and its acetate; 8-hydroxyquinoline sulfate; hydroxyzine and its pamoate and hydrochloride salts; ibuprofen; indomethacin; inositol; insulin; iodine; ipecac; iron; isosorbide and its mono- and dinitrates; isoxicam; ketamine; kaolin; ketoprofen; lactic acid; lanolin; lecithin; leuprolide acetate; lidocaine and its hydrochloride salt; lifinopril; liotrix; loperamide, loratadine; lovastatin; luteinizing hormore; LHRH (lutenizing hormone replacement hormone); magnesium carbonate, hydroxide, salicylate, and trisilicate; meclizine; mefenamic acid; meclofenamic acid; meclofenamate sodium; medroxyprogesterone acetate; methenamine mandelate; menthol; meperidine hydrochloride; metaproterenol sulfate; methscopolamine and its nitrates; methsergide and its maleate; methyl nicotinate; methyl salicylate; methyl cellulose; methsuximide; metoclopramide and its halides/hydrates; metronidazole; metoprotol tartrate; miconazole nitrate; mineral oil; minoxidil; morphine; naproxen and its alkali metal sodium salts; nifedipine; neomycin sulfate; niacin; niacinamide; nicotine; nicotinamide; nimesulide; nitroglycerine; nonoxynol-9; norethindrone and its acetate; nystatin; octoxynol; octoxynol-9; octyl dimethyl PABA; octyl methoxycinnamate; omega-3 polyunsaturated fatty acids; omeprazole; ondansetron and its hydrochloride; oxolinic acid; oxybenzone; oxtriphylline; para-aminobenzoic acid (PABA); padimate-O; paramethadione; pentastatin; peppermint oil; pentaerythritol tetranitrate; pentobarbital sodium; perphenazine; phenelzine sulfate; phenindamine and its tartrate; pheniramine maleate; phenobarbital; phenol; phenolphthalein; phenylephrine and its tannates and hydrochlorides; phenylpropanolamine; phenytoin; pirmenol; piroxicam and its salts; polymicin B sulfate; potassium chloride and nitrate; prazepam; procainamide hydrochloride; procaterol; promethazine and its hydrochloride; propoxyphene and its hydrochloride and napsylate; pramiracetin; pramoxine and its hydrochloride salt; prochlorperazine and its maleate; propanolol and its hydrochloride; promethazine and its hydrochloride; propanolol; pseudoephedrine and its sulfates and hydrochlorides; pyridoxine; pyrolamine and its hydrochlorides and tannates; quinapril; quinidine gluconate and sulfate; quinestrol; ralitoline; ranitadine; resorcinol; riboflavin; salicylic acid; scopolamine; sesame oil; shark liver oil; simethicone; sodium bicarbonate, citrate, and fluoride; sodium monofluorophosphate; sucralfate; sulfanethoxazole; sulfasalazine; sulfur; sumatriptan and its succinate; tacrine and its hydrochloride; theophylline; terfenadine; thiethylperazine and its maleate; timolol and its maleate; thioperidone; tramadol; trimetrexate; triazolam; tretinoin; tetracycline hydrochloride; tolmetin; tolnaftate; triclosan; trimethobenzamide and its hydrochloride; tripelennamine and its hydrochloride; tripolidine hydrochloride; undecylenic acid; vancomycin; verapamil HCI; vidaribine phosphate; vitamins A, B, C, D, B₁, B₂, B₆, B₁₂, E, and K; witch hazel; xylometazoline hydrochloride; zinc; zinc sulfate; zinc undecylenate. Active agents may further include, but are not limited to food acids; insoluble metal and mineral hydroxides, carbonates, oxides, polycarbophils, and salts thereof; adsorbates of active drugs on a magnesium trisilicate base and on a magnesium aluminum silicate base, and mixtures thereof. Mixtures and pharmaceutically acceptable salts of these and other actives can be used.

In one embodiment, the dosage forms coated with the dip coatings of the present invention provided for immediate release of the active ingredient, i.e. the dissolution of the dosage form conformed to USP specifications for immediate release tablets containing the particular active ingredient employed. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19 - 20 and 856 (1999).

We have unexpectedly found that the coatings formed by dipping substrates into the compositions of the present invention possessed excellent properties comparable to those possessed by gelatin coatings, e.g. crack resistance, hardness, thickness, color uniformity, smoothness, and gloss. The coatings of the present invention possessed a surface gloss of greater than 150, e.g. greater than 190 or greater than 210 when measured according to the method set forth in example 7 herein.

In addition, tablets dip coated with the compositions of the present invention were superior to tablets dip coated with conventional gelatin-based coatings in several important ways. First, tablets dip coated with the compositions of the present invention advantageously retained acceptable dissolution characteristics for the desired shelf-life and storage period at elevated temperature and humidity conditions. In particular, thehe cellulose-ether based compositions according to the present invention were also advantageously more resistant to microbial growth, which thereby enabled a longer shelf-life or use-life of the dipping solution as well as a reduction in manufacturing cost. Second, the sugar-thickened dipping dispersions according to the present invention beneficially employed a lower water content relative to that of gelatin-containing dispersions, which thereby enabled a shorter drying cycle time. Although the water content of the other dipping dispersions of the present invention may have been higher than that typically found in gelatin-based dipping solutions, the cellulose-ether based compositions of the present invention surprisingly required a shorter drying cycle time relative to that for gelatin-containing compositions. Third, the dried coatings comprised of the compositions of the present invention also surprisingly and advantageously contained fewer air bubbles relative to the amount present in dried, gelatin based dipping compositions. Fourth, unlike dip processing with gelatin-containing compositions, substrates may optionally be dipped in the solutions of the present invention at room temperature, which is economically more beneficial. Fifth, the dip coated compositions of the present invention possessed a higher degree of glossiness relative to similar coatings applied via spray coating methods known in the art. The dip coated compositions of the present invention also possessed a similar degree of glossiness relative to that possessed by gelatin-containing dip or enrobing coatings, which are currently viewed as the industry benchmark for high gloss coatings. See, e.g., United States Patent No. 6,274,162 (Typical gloss readings for standard, commercially available gel-dipped or gelatin enrobed tablets range from about 200 to 240 gloss units, gloss readings for standard, commercialy available sugar-coated medicaments range from 177 to 209 gloss units, and gloss readings for a new, high-gloss coating system range from about 148 to about 243 gloss units.).

We have further unexpectedly found that the addition of an effective amount of weight gain enhancer to a film forming composition comprised of film former and hydrocolloid not only significantly increased the resulting dry weight of the dip coating on a substrate, but it also improved the color uniformity of the coating.

The invention illustratively disclosed herein suitably may be practiced in the absence of any component, ingredient, or step which is not specifically disclosed herein. Several examples are set forth below to further illustrate the nature of the invention and the manner of carrying it out. However, the invention should not be considered as being limited to the details thereof.

### Comparative Example 1.) Preparation of Subcoating Dispersions

An aqueous dispersion containing the ingredients set forth in Table A was prepared by combining all of the ingredients in a beaker under ambient conditions.

**Table A: Aqueous Dispersion Subcoating Composition**

| **Ingredient** | **Part *** |
|---|---|
| HPMC (2910, 5 mPs) from Dow Chemical Company under the tradename, "Methocel E-5" | 20 |
| Castor oil | 1 |
| Water | 241.5 |
| Total Coating Solution | 262.5 |
| % solids in coating solution | 8% |

| | |
|---|---|
| * expressed in terms of part by weight unless otherwise noted | |

Additional aqueous dispersions containing the ingredients in Table B were similarly prepared:

**Table B: Aqueous Dispersion Subcoating Compositions**

| **Ingredient** | **Ex 1A**** | **Ex 1B** | **Ex 1C** | **Ex 1D** | **Ex 1E** |
|---|---|---|---|---|---|
| HPMC 2910, 5 mPs | 20 | 40 | 40 | 28 | 28 |
| Castor oil | 1 | 0 | 0 | 0 | 0 |
| water | 212.3 | 566.67 | 566.67 | 566.67 | 566.67 |
| maltodextrin | 0 | 53 | 53 | 67 | 67 |
| PEG 400 | 0 | 7 | 7 | 5 | 5 |
| Hydroxyethylcellulose* | 0 | 0 | 0 | 0 | 0 |
| Total coating solution | 233.3 | 666.67 | 666.67 | 666.67 | 666.67 |
| Wt % solids in coating solution | 9% | 15% | 15 | 15 | 15 |

| | | | | | |
|---|---|---|---|---|---|
| * Available from Aqualon, under the tradename, "Natrosol 250L" ** all values expressed in terms of parts by weight unless otherwise noted | | | | | |

Additional aqueous dispersions containing the ingredients in Table C were similarly prepared:

**Table C: Aqueous Dispersion Subcoating Compositions**

| **Ingredient** | **Ex 1F**** | **Ex 1G** | **Ex 1H** |
|---|---|---|---|
| water | 566.67 | 566.67 | 690.4 |
| maltodextrin | 71 | 71 | 0 |
| Castor oil | 0 | 0 | 0.13 |
| HPMC (1910, 5mPas) | 0 | 0 | 32.4 |
| PEG 400 | 5 | 5 | 0 |
| Hydroxyethylcellulose* | 24 | 24 | 0 |
| Total coating solution | 666.67 | 666.67 | 722.9 |
| Wt % solids in coating solution | 15% | 15% | 4.5% |

| | | | |
|---|---|---|---|
| * Available from Aqualon, under the tradename, "Natrosol 250L" ** all values expressed in terms of parts by weight unless otherwise noted | | | |

### Comparative Example 2.) Preparation of Subcoated Tablets

Compressed tablets were prepared in accordance with the procedure set forth in Example 1 of United States Patent No. 5,658,589 ("'589 Patent").

The dispersion of Example 1 was then applied onto the compressed tablets via spraying in accordance with the procedure set forth in the examples of the '589 Patent . As shown in Table D below, the dried subcoated tablets possessed an average 2% to 4% weight gain relative to the weight of the subcoating-free tablets.

This process was repeated with additional compressed tablets, but with the substitution of each, respective subcoating dispersion produced in Example 1A to 1H for that of Example 1. The percentage weight gain of the dried subcoated tablets are set forth below in Table D:

**Table D: % Weight Gain of Dried Subcoated Tablets**

| **Example Number** | **% Weight Gain** |
|---|---|
| 1A | 2 |
| 1B | 2 |
| 1C | 4 |
| 1D | 2 |
| 1E | 4 |
| 1F | 2 |
| 1G | 4 |
| 1H | 4 |

### Comparative Example 3) Preparation of HPMC Coated Tablets

Aqueous HPMC dipping solutions containing the ingredients set forth in Table E were prepared:

**Table E: Composition of HPMC Dipping Solutions**

| **Ingredient** | **Ex 3A *(g)** | **Ex 3B (g)** | **Ex 3C (g)** | **Ex 3D (%)** | **Ex 3E (%)** | **Ex 3F (%)** |
|---|---|---|---|---|---|---|
| HPMC E5 | 32.5 | 0 | 32.5 | 10 | 11 | 14 |
| Water | 200 | 200 | 200 | 89.89 | 88.879 | 85.85 |
| HPMC (2910, 15mPs) | 0 | 20 | 0 | | 0 | 0 |
| Xanthan gum | 0 | 0 | 0 | 0.11 | 0.121 | 0.15 |
| PEG 400 | 0 | 0 | 8 | 0 | 0 | 0 |
| % (wt.) solids in dipping solution | 14 | 9 | 17 | 10.11 | 11.121 | 14.15 |

| | | | | | | |
|---|---|---|---|---|---|---|
| * all values expressed in terms of weight (g) unless otherwise noted | | | | | | |

Example 3A: Preparation Of Dipping Solution of Example 3A: HPMC was dispersed into 200 ml of deionized water at a temperature of 70 °C. After adding about 1 wt % FD&C blue dye thereto, the solution was mixed until homogeneous. The solution was then cooled to a temperature of about 22 °C.

Example 3B: Preparation of Dipping Solution of Example 3B: The procedure of Example 3A was repeated, but with substitution of HPMC (2910, 15mPs) for the HPMC E5.

Example 3C: Preparation Of Dipping Solution of Example 3C: HPMC was dispersed into 200 ml of deionized water at a temperature of 70 °C. After adding the PEG 400 thereto, the solution was mixed until homogeneous. The solution was then cooled to a temperature of about 22 °C.

Example 3D: Preparation Of Dipping Solution of Example 3D: HPMC and xanthan gum were added to purified water at a temperature of 80 °C until the powder was dispersed. After discontinuing the heat, the solution was divided into two parts. 4.35 wt. % of a yellow color dispersion available from Colorcon, Inc. under the tradename, "Opatint Yellow DD-2115" was added to the first part and mixed at a low speed until dispersed. 5.8% of a green color dispersion available from Colorcon, Inc. under the tradename, "Opatint Green DD-11000" was added to the second part and mixed at a low speed until dispersed. The two dispersed solutions were then stored under ambient conditions for about 12 hours.

Example 3E: Preparation Of Dipping Solution of Example 3E: The procedure of Example 3D was repeated, but using the components of Example 3E.

Example 3F: Preparation Of Dipping Solution of Example 3F: The procedure of Example 3D was repeated, but using the components of Example 3F.

Example 3G: Preparation of Hand-Dipped Dip Coated Tablets: The subcoated tablets prepared in accordance with Example 2 using the subcoating produced in Example 1H were hand-dipped into the dipping solutions of Example 3A for a dwell time of 1 second, removed from the dipping solution, then dried under ambient conditions.

This procedure was repeated, but with substitution of the dipping solutions of Examples 3B and 3C, respectively, for the dipping solution of Example 3A.

An observation of the resulting coatings showed the following:
Tablets Coated with Coating of Ex. 3A: The coatings were smooth, hard, and shiny, and had no bubbles or cracking. However, the coatings were non-uniform and thin, with land areas not well-covered. Upon exposure to ambient conditions for a six month period, no cracks were seen in the coatings.
Tablets Coated with Coating of Ex. 3B: The coating were shiny, with few bubbles and no cracking. The coatings were more uniform and rough relative to those of Example 3A. The coatings were also somewhat tacky and thin, with land areas not well-covered. Upon exposure to ambient conditions for a six month period, no cracks were seen in the coatings.
Tablets Coated with Coating of Ex. 3C: The coatings were shiny with few bubbles and no cracking. The coatings were more uniform and rough relative to those of Example 3A. The coatings were also somewhat tacky and thin, with land areas not well-covered. Upon exposure to ambient conditions for a six month period, no cracks were seen in the coatings.

Example 3H: Preparation of Production Scale Dipped Tablets: Additional subcoated tablets prepared in accordance with Example 2 using the subcoating produced in Example 1H were coated with the resulting dipping solution of Examples 3D using a commercial grade gel-dipping machine in accordance with the procedure described in United States Patent No. 4,820,524.

This procedure was repeated, but with substitution of the dipping solutions of Examples 3E and 3F, respectively, for the dipping solution of Example 3D.

The average percentage weight gain of the dried dipped coatings were as set forth in Table F:

**Table F:Weight Gain of Dried Dip Coating**

| **Example** | **% Wt. Gain of Dried Coating*** |
|---|---|
| Ex. 3D | 0.75 - 2.26 |
| Ex. 3E | 1.9 - 3.52 |
| Ex. 3F | 3.2 - 5.8 |

| | |
|---|---|
| * Relative to weight of dried subcoating and core | |

This example showed that the addition of xanthan gum to the HPMC dipping solution provided a viscosity enhancement to the dip coating, and thus an increased weight gain of the dip coating on the tablets.

### Example 3I: Preparation of Dipping Solution of Example 3I

The procedure of Example 3D was repeated, but using the components of Example 3I, as set forth in Table M:

**Table M: Composition of HPMC Dipping Solutions**

| **Ingredient** | **Ex 3I *(g)** | **Ex 3J (g)** |
|---|---|---|
| HPMC E5 | 14 | 12 |
| Water | 85.89 | 87.88 |
| HPMC (2910, 15mPs) | 0 | 0 |
| Xanthan gum | 0.11 | 0.12 |
| PEG 400 | 0 | 0 |
| % (wt.) solids in dipping solution | 14.11 | 12.12 |

| | | |
|---|---|---|
| * all values expressed in terms of weight (g) unless otherwise noted | | |

### Example 3J: Preparation of Dipping Solution of Example 3J

The procedure of Example 3D was repeated, but using the components of Example 3J, as set forth in Table M above.

### Example 4) Preparation of Pre-gelatinized Starch-Containing Dip Coating Solutions

Dipping solutions comprised of the components set forth in Table G were prepared by dispersing 75 g of the modified waxy maize starch into 200 ml of water under ambient conditions with mixing:

**Table G: Pre-gelatinized starch-containing Dipping solutions**

| **Component/Other** | **Example 4A*** | **Example 4B** |
|---|---|---|
| Modified waxy maize starch (Purity@ Gum 59) | 75 | 125 |
| water | 200 | 200 |
| Total weight of solution | 275 | 325 |
| Wt % solids in dipping solution | 27 | 39 |

| | | |
|---|---|---|
| * all values expressed in terms of weight (g) unless otherwise noted | | |

Dipping solutions comprised of the components set forth in Table H below were prepared by dispersing all of the components into 200 ml of water under ambient conditions with mixing until the resulting solution was clear.

**Table H: Pre-gelatinized starch-containing Dipping solutions With Simethicone of Example 4C**

| ***Component*** | ***Tradename*** | ***Suppiler*** | ***Amount used **** |
|---|---|---|---|
| Modified waxy maize starch | Purity ® Gum 59 | National Starch & Chemical Co. | 125 |
| Simethicone | Antifoam® | | 2 |
| Colloidal silicone dioxide | Aerosil ® A200 | | 6 |
| Glycerin | --- | --- | 63.5 |
| Sucrose | --- | --- | 38 |
| colorant | Opatint® | | 6.9 |
| water | --- | --- | 200 |
| Total solids | | | 241.4 |
| TOTAL solution (w/55% solids) | | | 441.1 |

| | | | |
|---|---|---|---|
| * all values expressed in terms of weight (mg) unless otherwise noted | | | |

Each side of the subcoated tablets prepared in accordance with Example 2 using the subcoating produced in Example 1H were hand-dipped into the dipping solution of Example 4A for a dwell time of about 1 second, pulled up, then dried under ambient conditions.

This procedure was repeated, but with substitution of the dipping solution of Example 4B for the dipping solution of Example 4A and with about a 3 day period between the completion of production of the dipping solution and the commencement of dip coating process.
This procedure was further repeated, but with substitution of the dipping solutions of Example 4C for the dipping solution of Example 4A and with about a 12 hour period between the completion of production of the dipping solution and the commencement of dip coating process.

An observation of the resulting coatings showed the following:
Tablets Coated With Dipping Solution of Ex. 4A: The coatings were very shiny, hard, smooth, even, and not tacky or cracked. However, the coatings were too thin, and land areas were not covered. No cracking after exposure to ambient conditions for a period of 6 months.
Tablets Coated With Dipping Solution of Ex. 4B: The coatings were smooth and shiny. Initially the land areas were covered; however, the coatings cracked after exposure to ambient conditions for a period of 6 months.
Tablets Coated With Dipping Solution of Ex. 4C: The coatings possessed excellent shine and cover, and were smooth with no cracks. No cracking after exposure to ambient conditions for a period of 2 months.

### Example 5) Preparation of Pre-Gelatinized Starch-Containing Dip Coating Solutions

The procedure set forth in Example 4C is repeated, but without the inclusion of simethicone. Prior to coating the substrate, the solution is exposed to a vacuum pressure of 5 inches Hg in order to remove substantially all of the visible bubbles from the solution. The resulting coating possesses excellent shine and cover, and is smooth with no cracks.

### Comparative Example 6) Effect of Simethicone on Coating Weight Gain

The following dip coating solutions set forth in Table I were prepared to illustrate the effect of simethicone as a weight gain enhancer. Amounts are percent based on the total weight of coating solution.

**Table I Dip Coating Solutions**

| Ingredient | 6A | 6B | 6C | 6D | 6E |
|---|---|---|---|---|---|
| HPMC 2910, 5mPs | 12 | 12 | 12 | 12 | 12 |
| Xanthan Gum | 1 | 1 | 1 | 1 | 1 |
| Simethicone | 0 | 0.035 | 0.07 | 0.14 | 0.25 |
| Yellow color dispersion*** | 6 | 6 | 6 | 6 | 6 |
| Water | 81 | 80.965 | 80.93 | 80.86 | 80.75 |

| | | | | | |
|---|---|---|---|---|---|
| *** Yellow color dispersion was "Opatint"® No. DD2125 obtained from Colorcon, Inc. | | | | | |

Dipping solutions A through E, above, were prepared in the following manner: Purified water was heated to about 35°C. HPMC and xanthan gum were added while mixing using a laboratory scale electric mixer (Janke and Kunkel, IKA Labortechnik, Staufen, Germany) with propeller blade at approximately 1000 rpm until the powders appeared uniformly dispersed. Heating was discontinued, and the resulting dispersion was allowed to stand overnight at room temperature. Simethicone and yellow color dispersion were then added with mixing at approximately 500 rpm.

Subcoated cores, prepared according to the method of example 1A, were pre-weighed, then dipped in solutions A, B, C, D, and E, above for a dwell time of about 2 seconds, pulled up, then dried at ambient conditions (about 22 °C). The cores were dipped simultaneously in sets of 7. Three separate sets of seven cores were dipped in each solution A through E. The average weight gain was determined from the triplicate sets of dipped cores from each coating solution.

Resulting weight gains were as follows in Table J:

**Table J - Average Weight Gain**

| Dipping Solution | 6A | 6B | 6C | 6D | 6E |
|---|---|---|---|---|---|
| Average weight gain from dip coat (mg/tablet) | 13.3 | 20.8 | 22.3 | 23.7 | 19.1 |

### Example 7) Surface Gloss Measurement of Coated Tablets

Tablets made according to the preceding examples were tested for surface gloss using an instrument available from TriCor Systems Inc. (Elgin, IL) under the tradename, " Tri-Cor Model 805A/806H Surface Analysis System" and generally in accordance with the procedure described in "TriCor Systems WGLOSS 3.4 Model 805A/806H Surface Analysis System Reference Manual" (1996),which is incorporated by reference herein, except as modified below,

This instrument utilized a CCD camera detector, employed a flat diffuse light source, compared tablet samples to a reference standard, and determined average gloss values at a 60 degree incident angle. During its operation, the instrument generated a greyscale image, wherein the occurrence of brighter pixels indicated the presence of more gloss at that given location.

The instrument also incorporated software that utilized a grouping method to quantify gloss, i.e., pixels with similar brightness were grouped together for averaging purposes.

The "percent full scale" or "percent ideal" setting (also referred to as the "percent sample group" setting), was specified by the user to designate the portion of the brightest pixels above the threshold that will be considered as one group and averaged within that group. "Threshold", as used herein, is defined as the maximum gloss value that will not be included in the average gloss value calculation. Thus, the background, or the non-glossy areas of a sample were excluded from the average gloss value calculations. The method disclosed in K. Fegley and C. Vesey, "The Effect of Tablet Shape on the Perception of High Gloss Film Coating Systems", which is available at www.colorcon.com as of 18 March, 2002 and incorporated by reference herein, was used in order to minimize the effects resulting from different tablet shapes, and thus report a metric that was comparable across the industry.(Selected the 50% sample group setting as the setting which best approximated analogous data from tablet surface roughness measurements.).

After initially calibrating the instrument using a calibration reference plate (190-228; 294 degree standard; no mask, rotation 0, depth 0), a standard surface gloss measurement was then created using gel-coated caplets available from McNEIL-PPC, Inc. under the tradename, "Extra Strength Tylenol Gelcaps." The average gloss value for a sample of 112 of such gel-coated caplets was then determined, while employing the 25 mm full view mask (190-280), and configuring the instrument to the following settings:
Rotation: 0
Depth: 0.25 inches
Gloss Threshold: 95
% Full Scale: 50%
Index of Refraction: 1.57

The average surface gloss value for the reference standard was determined to be 269, using the 50% ideal (50% full scale) setting.

Samples of coated tablets prepared according to the preceding examples were then tested in accordance with the same procedure. The surface gloss values at the 50% ideal setting that were obtained are summarized in Table K below.

**Table K: Gloss values of coated tablets**

| **Example No.** | **3D** | **3I** | **3J** | **4C** | **6B** |
|---|---|---|---|---|---|
| Type of coating | dipped | dipped | dipped | poured film | dipped |
| No. of tablets tested | 48 | 48 | 51 | plate | 3 |
| Gloss Value(% ideal at 50) | 234 | 247 | 229 | 259 | 221 |

Additional samples of other, commercially available gel coated tablets were also tested in accordance with the same procedure and compared to the same standard. The results are summarized in Table L below.

**Table L: Gloss values of commercially available coated tablets**

| **Product** | **Motrin IB* Caplet (white)** | **Excedrin**Aspirin free Caplets (red)** | **Excedrin**Migraine Geltab (green side)** | **Excedrin**Migraine Geltab (white side)** | **Extra Strength Tylenol Geltabs* (yellow side)** | **Extra Strength Tylenol Geltabs* (red side)** |
|---|---|---|---|---|---|---|
| Type of coating | sprayed film | sprayed film | gelatin enrobed | gelatin enrobed | dipped | dipped |
| No. of tablets tested | 41 | 40 | 10 | 10 | 112 | 112 |
| Gloss Value(% ideal at 50) | 125 | 119 | 270 | 264 | 268 | 268 |

| | | | | | | |
|---|---|---|---|---|---|---|
| * Available from McNEIL-PPC, Inc. ** Available from Bristol-Myers, Squibb, Inc. | | | | | | |

This Example showed that the tablets coated with the compositions of the present invention possessed a high surface gloss value that either was comparable to or exceeded that possessed by commercially -available gelatin coated tablets. In contrast, typical sprayed films possessed a substantially lower surface gloss, e.g. 119 to 125 in this Example.

### Comparative Example 8: Preparation of Coated tablets

### Example 8A: Preparation of Tablets Spray Coated With Opadry® II Subcoating

122.8 kg (18% w/w) of a prepared blend containing HPMC 2910-6cP, maltodextrin, HPMC2910-3cP, HPMC2910-50cP, and PEG-400 (commercially available from Colorcon Inc., West Point, PA as "Opadry® II") was added with mixing into 559.7 kg (82% w/w) of 35°C purified water in a conventional pressure pot, and mixed with an air-driven propeller-type Lightnin mixer at a speed of 500 rpm. After the powder was completely added, the dispersion was mixed at 500 rpm for 2 hours, then allowed to stand without mixing at ambient conditions for 12 hours.

The resulting film coating dispersion was then applied onto compressed acetaminophen tablets, which were prepared in accordance with the procedure set forth in Example 1 of US Patent No. 5,658,589 ("'589 Patent"), via spraying in accordance with the procedure set forth in the examples of the '589 patent. The resulting spray-coated tablets possessed a 4% weight gain relative to the weight of the uncoated tablet cores.

### Examples 8B: Preparation of Tablets Spray Coated with HPMC / Castor Oil Subcoating

88.4 kg (9% w/w) of hydroxypropyl methylcellulose 2910, 5mPs and 0.347 kg (0.04% w/w) of castor Oil were mixed into 593.8 kg (91% w/w) of purified water at 35°C in a tank with mixer (Lee Industries) at a speed of 1750 rpm. After the powder was completely added, the mixer speed was increased to 3500 rpm for 15 minutes. The mixer speed was then reduced to 1750 rpm while the pressure was reduced to 15 inches of water for 2 hours to deaerate the dispersion.

The resulting film coating dispersion was then applied onto the compressed acetaminophen tablets of Example 8A via spraying in accordance with the procedure set forth above in Example 8A . The resulting spray coated tablets possessed a 4% weight gain relative to the weight of the uncoated tablet cores.

### Example 8C: Preparation of Tablets Dip Coated with HPMC/Castor Oil dipping solutions

A dipping solution comprised of the components set forth in Table M below was produced:

**Table M: HPMC/Castor Oil Clear Dipping Solutions**

| Example | A&B | C&D | E&F |
|---|---|---|---|
| HPMC 2910 5mPs | 9% | 13% | 13% |
| Castor Oil | 0.04% | 0.05% | 0.05% |
| Purified Water | 90.96% | 86.95% | 86.95% |

Purified water was heated to 80° C, then added to a Lee jacketed mix tank while mixing at a speed of 1750 rpm. After HPMC 2910, 5mPs and castor oil were added thereto with mixing, the mixer speed was increased to 3500 rpm for 15 minutes. The mixer speed was then reduced to 1750 rpm while the temperature of the dispersion was reduced to 35 °C and the pressure was reduced to 15 inches water for deaeration. After mixing the dispersion for 2 hours, the resulting dispersion remained under constant pressure conditions for an additional 3 hours without mixing.

The colorant of Example 8C-a was then added to 96 kg of the resulting clear dipping solutions with mixing at a 1750 rpm speed in the amounts set forth in Table N below :

**Table N: HPMC/Castor Oil Colored Dipping Solutions**

| ***Example*** | ***8C-a*** | ***8C-b*** | ***8C-c*** | ***8C-d*** | ***8C-e*** | ***8C-f*** |
|---|---|---|---|---|---|---|
| Colorant | Opatint (DD-1761) | Opatint (DD-2125) | Opatint (DD-1761) | Opatint (DD-2125) | Opatint (DD-10516 | Opatint (DD-18000) |
| Amount of colorant (kg) | 2.700 | 2.570 | 2.700 | 2.570 | 4.072 | 2.175 |
| Color | red | yellow | red | yellow | blue | white |
| Visc/Temp | 490 cps @40C | 518 cps @40C | 612cps @30C | 457cps @30C | 351cps @40C | 319cps @40C |
| Dipping Temp | 40C | 40C | 30C | 30C | 40C | 40C |
| Weight Gain in dipping (mg/tablet) | 16 | 16 | 26 | 26 | 20 | 20 |
| Gloss | 229 | 229 | 249 | 228 | 238 | 233 |

This procedure was independently repeated for each of the colorants set forth above in Table N.

Subcoated tablets, which were prepared in accordance with the procedure set forth above in Example 8A, were dip-coated with the dip-coating solution prepared in accordance with Example 8C-a and 8C-b using a commercial grade gel-dipping machine and in accordance with the procedure described in United States Patent No. 4,820,524, using the dipping solution temperatures reported in the table above. This procedure was independently repeated on subcoated tablets, which were prepared in accordance with the procedure set forth above in Example 8B, for each of the colored dipping solutions 8C-c through 8C-f in Table N above.

A visual comparison of the dip-coated tablets prepared according to examples 8C-a and 8C-b with those prepared in accordance with Examples 8C-c through 8C-f revealed that the former did not possess complete coating coverage around the edges of the tablets. By contrast, the dip-coated tablets prepared according to examples 8C-c through 8C-f possessed a superior good coating coverage around the tablet edges. This indicated that a weight gain of 16 mg per gelcap (such as that produced by the 9% HPMC formula of examples 8C-a and 8C-b) was insufficient for the HPMC/Castor Oil dipping formula, while a weight gain of 20 to 26 mg per gelcap/geltab (such as that produced by the 13% HPMC formula of examples 8C-c through 8C-f) provided good coverage.

In addition, a visual comparison of the HPMC/Castor Oil dip-coated tablets of Examples 8C-c through 8C-f and the HPMC/Xanthan Gum dip-coated tablets of Examples 3I and 3J indicated that the former possessed superior gloss and surface smoothness. The superior gloss and smoothness were likely attributed to the inclusion of castor oil in the dip coating.

### Comparative Example 9: Preparation of Tablets Dip Coated with HPMC/Maltodextrin/PEG dipping solutions

143.3 kg (21% w/w) of the Opadry® II blend of Example 8A was added into 539.2 kg (79% w/w) of 35°C purified water while mixing at a speed of 3500 rpm for 15 minutes. The mixer speed was then decreased to 1750 rpm, and the tank evacuated to 30 PSIA to deaerate the solution for 5 hours. 2.70 kg of Colorant (Opatint® Red DD-1761, from Colorcon Inc.) was then added to 96 kg of the clear dipping solution while mixing at a speed of 1750 rpm. 2.570 kg of Colorant (Opatint® Yellow DD-2125, from Colorcon Inc.) was then added to a second 96 kg portion of the clear dipping solution while mixing at a speed of 1750 rpm until dispersed.

Subcoated tablets, which were prepared in accordance with the procedure and materials set forth above in Example 8B, were dip-coated with the dip-coating solution prepared in accordance with this Example using a commercial grade gel-dipping machine and in accordance with the procedure described in United States Patent No. 4,820,524, which is incorporated by reference herein, using a dipping solution temperature of 30°C. The viscosity of the dipping solutions was 607 cPs at 30°C for the yellow solution, and 677 cPs at 30°C for the red solution. An average weight gain of about 27 mg/gelcap was obtained.

Seventy-two (72) dipped gel caps produced in accordance with this Example were tested for surface gloss in accordance with the procedure set forth in Example 7. The average surface gloss for these dipped gelcaps was 258 gloss units.

### Comparative Example 10: Preparation of Tablets Dip Coated with HPMC/Carrageenan dipping solutions

88.4 kg (13% w/w) of HPMC 2910-5 mPs, 0.347 kg of Castor Oil (0.05% w/w), and 0.68 kg (0.1% w/w) of kappa Carrageenan-911 were added into a tank containing 590 kg (87% w/w) of 80°C purified water while mixing at a speed of 1750 rpm. After the addition was complete, the mixer speed was increased to 3500 rpm for 15 minutes. The mixer speed was then decreased to 1750 rpm, and the tank evacuated to 15 inches of water to deaerate the solution for 2 hours. Mixing was then stopped, and the dispersion was allowed to stand at constant pressure for an additional 3 hours. 2.175 kg of Colorant (Opatint® White DD-18000, from Colorcon Inc.) was then added to 96 kg of the clear dipping solution while mixing at a speed of 1750 rpm. 4.072 kg of Colorant (Opatint® Blue DD-10516, from Colorcon Inc.) was then added to a second 96 kg portion of the clear dipping solution while mixing at a speed of 1750 rpm until dispersed.

Subcoated tablets, which were prepared in accordance with the procedure and materials set forth above in Example 8B, were dip-coated with the dip-coating solution prepared in accordance with this Example using a commercial grade gel-dipping machine and in accordance with the procedure described in United States Patent No. 4,820,524, which is incorporated by reference herein, using a dipping solution temperature of 40°C. An average weight gain of about 20 mg/gelcap was obtained.

Eighty-eight (88) dipped gel caps produced in accordance with this Example were tested for surface gloss in accordance with the procedure set forth in Example 7. The average surface gloss for these dipped geltabs was 232 gloss units.

### Example 11: Preparation of tablets dip coated with hydroxypropyl starch containing dipping solution.

92.5 g of hydroxypropylated modified dent corn starch ("Pure-Cote B790", Grain Processing Corporation) was added to a tank containing 300g of 80°C purified water while mixing with a Kika-Werk RW 20 DZM motor fitted with a 4 cm propeller blade at a speed of 650 rpm for 30 minutes. 7.5 g of Gellan Gum ("Kelco gel", Kelco) was then added thereto with constant mixing for 15 min. 2.6g of colorant ("Opatint Red DD-1761" Colorcon) was then added into the 80C solution with constant mixing for 15 min. The resulting solution contained the ingredients set forth below in Table O:

**Table O: Hydroxypropyl Starch Based Dipping Solutions**

| ***Component*** | Trade name | ***Supplier*** | ***Amount Used**** |
|---|---|---|---|
| | | | |
| Hydroxypropyl Starch | Pure-Cote B790 | Grain Processing Corporation | 92.5 |
| Gellan Gum | Kelcogel | Kelco | 7.5 |
| Colorant | Opatint Red | Colorcon | 2.6 |
| Water | N/A | N/A | 300 |

| | | | |
|---|---|---|---|
| *All values expressed in terms of weight (grams) unless otherwise stated | | | |

Subcoated tablets, which were prepared in accordance with the procedure and materials set forth above in Example 8B, were dipped by hand in the resulting solution at 22°C, then dried at ambient conditions for 18 hours. An average weight gain of about 47 mg/gelcap was obtained.

Dipped gel caps produced in accordance with this Example were then tested for surface gloss in accordance with the procedure set forth in Example 7. The average surface gloss for these dipped geltabs was 229 gloss units.

## Claims

1. A pharmaceutical dosage form comprising a core and a dip-coated coating layer; said coating layer substantially covering said core, **characterised in that** said coating layer is comprised of, based upon the total dry weight of the coating layer:
a) from 40 percent to 60 percent of a film former selected from the group consisting of a waxy maize starch, tapioca dextrin, a derivative of a waxy maize starch, a derivative of tapioca dextrin and mixtures thereof;
b) from 5 percent to 25 percent thickener selected from the group consisting of sucrose, dextrose, fructose and mixtures thereof; and
c) from 15 percent to 30 percent plasticizer,
wherein the coating layer possesses a surface gloss of at least 150.

2. A pharmaceutical dosage form comprising a core and a dip-coated coating layer; said coating layer substantially covering said core, **characterised in that** said coating layer is comprised of, based upon the total dry weight of the coating layer:
a) from 25 percent to 80 percent hydroxypropyl starch film former;
b) from 0.10 percent to 33 percent thickener selected from the group consisting of kappa carrageenan, iota carrageenan, maltodextrin, gellan gum, agar, gelling starch and derivatives and mixtures thereof; and
c) 11 percent to 60 percent of a plasticizer,
wherein the coating layer possesses a surface gloss of at least 150 and has a thickness of 40 microns to 400 microns.

3. The dosage form of claim 2, wherein the coating layer is comprised of, based upon the total dry weight of the coating layer,
a) from 25 percent to 80 percent of hydroxypropyl starch;
b) from 0.1 percent to 33 percent of a thickener selected from the group consisting of kappa carrageenan, iota carrageenan, maltodextrin, gellan gum, agar, gelling starch and mixtures thereof; and
c) from 11 percent to 60 percent of a plasticizer selected from the group consisting of glycerin, propylene glycol, polyethylene glycol, sugar alcohols and mixtures thereof.

4. The dosage form of claim 1 wherein the plasticizer is glycerin and the thickener is sucrose.

5. The dosage form of claim 1, wherein the coating layer is comprised of, based upon the total dry weight of the coating layer,
a) from 40 percent to 60 percent of a film former selected from the group consisting of a waxy maize starch, tapioca dextrin, a derivative of a waxy maize starch, a derivative of tapioca dextrin and mixtures thereof;
b) from 10 percent to 20 percent of sucrose; and
c) from 20 percent to 25 percent of glycerin.

6. A pharmaceutical dosage form according to any one of the preceding claims additionally comprising a subcoating substantially covering said core, and a coating layer substantially covering said subcoating.

7. The dosage form of any one of claims 1 to 6, comprising an effective amount of a pharmaceutical active ingredient, wherein said dosage form meets USP dissolution requirements for immediate release forms of said pharmaceutical active ingredient.

8. A simulated capsule-like medicament comprising:
a. a core having a first and a second end,
b. a first coating layer having a first color provided on said first end of said core;
c. a second coating layer having a second color on said second end of said core, said first color is different than said second color;
**characterised in that** at least one of said first coating layer and second coating layer is the coating layer as described in any one of claims 1 to 5.

9. A method of making coated tablets from an aqueous dispersion comprising the coating layer as described in any one of claims 1 to 5 comprising dip coating tablets in the aqueous dispersion.

## Patentansprüche

1. Pharmazeutische Dosierungsform, umfassend einen Kern und eine tauchbeschichtete Überzugsschicht; wobei die Überzugsschicht den Kern im Wesentlichen bedeckt, **dadurch gekennzeichnet, dass** die Überzugsschicht, bezogen auf das Gesamttrockengewicht der Überzugsschicht,
a) 40 Prozent bis 60 Prozent eines Filmbildners, ausgewählt aus der Gruppe, bestehend aus einer wachsartigen Maisstärke, Tapiokadextrin, einem Derivat einer wachsartigen Maisstärke, einem Derivat von Tapiokadextrin und Gemischen davon;
b) 5 Prozent bis 25 Prozent Verdickungsmittel, ausgewählt aus der Gruppe, bestehend aus Sucrose, Dextrose, Fructose und Gemischen davon; und
c) 15 Prozent bis 30 Prozent Weichmacher
umfasst, wobei die Überzugsschicht einen Oberflächenglanz von wenigstens 150 aufweist.

2. Pharmazeutische Dosierungsform, umfassend einen Kern und eine tauchbeschichtete Überzugsschicht; wobei die Überzugsschicht den Kern im Wesentlichen bedeckt, **dadurch gekennzeichnet, dass** die Überzugsschicht, bezogen auf das Gesamttrockengewicht der Überzugsschicht,
a) 25 Prozent bis 80 Prozent Hydroxypropylstärke-Filmbildner;
b) 0,10 Prozent bis 33 Prozent Verdickungsmittel, ausgewählt aus der Gruppe, bestehend aus kappa-Karrageenan, iota-Karrageenan, Maltodextrin, Gellangummi, Agar, gelierende Stärke und Derivaten und Gemischen davon; und
c) 11 Prozent bis 60 Prozent Weichmacher
umfasst, wobei die Überzugsschicht einen Oberflächenglanz von wenigstens 150 aufweist und eine Dicke von 40 Mikrometer bis 400 Mikrometer aufweist.

3. Dosierungsform gemäß Anspruch 2, wobei die Überzugsschicht, bezogen auf das Gesamttrockengewicht der Überzugsschicht,
a) 25 Prozent bis 80 Prozent Hydroxypropylstärke;
b) 0,10 Prozent bis 33 Prozent eines Verdickungsmittels, ausgewählt aus der Gruppe, bestehend aus kappa-Karrageenan, iota-Karrageenan, Maltodextrin, Gellangummi, Agar, gelierende Stärke und Derivaten und Gemischen davon; und
c) 11 Prozent bis 60 Prozent eines Weichmachers, ausgewählt aus der Gruppe, bestehend aus Glycerin, Propylenglycol, Polyethylenglycol, Zuckeralkoholen und Gemischen davon
umfasst.

4. Dosierungsform gemäß Anspruch 1, wobei der Weichmacher Glycerin ist und das Verdickungsmittel Sucrose ist.

5. Dosierungsform gemäß Anspruch 1, wobei die Überzugsschicht, bezogen auf das Gesamttrockengewicht der Überzugsschicht,
a) 40 Prozent bis 60 Prozent eines Filmbildners, ausgewählt aus der Gruppe, bestehend aus einer wachsartigen Maisstärke, Tapiokadextrin, einem Derivat einer wachsartigen Maisstärke, einem Derivat von Tapiokadextrin und Gemischen davon;
b) 10 Prozent bis 20 Prozent Sucrose; und
c) 20 Prozent bis 25 Prozent Glycerin
umfasst.

6. Pharmazeutische Dosierungsform gemäß einem der vorstehenden Ansprüche, zusätzlich umfassend eine Unterbeschichtung, die den Kern im Wesentlichen bedeckt, und eine Überzugsschicht, die die Unterbeschichtung im Wesentlichen bedeckt.

7. Dosierungsform gemäß einem der Ansprüche 1 bis 6, umfassend eine wirksame Menge eines pharmazeutischen Wirkstoffs, wobei die Dosierungsform den USP-Auflösungsmaßgaben für Formen mit sofortiger Freisetzung des pharmazeutischen Wirkstoffs entspricht.

8. Simuliert kapselähnliches Medikament, umfassend:
a. einen Kern mit einem ersten und einem zweiten Ende,
b. eine erste Überzugsschicht mit einer ersten Farbe auf dem ersten Ende des Kerns;
c. eine zweite Überzugsschicht mit einer zweiten Farbe auf dem zweiten Ende des Kerns, wobei die erste Farbe von der zweiten Farbe verschieden ist;
**dadurch gekennzeichnet, dass** wenigstens eine von der ersten Überzugsschicht und der zweiten Überzugsschicht die Überzugsschicht gemäß einem der Ansprüche 1 bis 5 ist.

9. Verfahren zum Herstellen überzogener Tabletten aus einer wässrigen Dispersion, umfassend die Überzugsschicht gemäß einem der Ansprüche 1 bis 5, umfassend Tauchüberziehen von Tabletten in der wässrigen Dispersion.

## Revendications

1. Forme de dosage pharmaceutique comprenant un coeur et une couche d'enrobage à trempage ; ladite couche d'enrobage couvrant sensiblement ledit coeur, **caractérisée en ce que** ladite couche d'enrobage est composée, sur la base du poids sec total de la couche d'enrobage :
a) de 40 pour cent à 60 pour cent d'un agent filmogène choisi dans le groupe constitué d'amidon de maïs waxy, de dextrine de tapioca, de dérivé d'un amidon de maïs waxy, de dérivé de dextrine de tapioca et de mélanges de ceux-ci ;
b) de 5 pour cent à 25 pour cent d'épaississant choisi dans le groupe constitué de saccharose, de dextrose, de fructose et de mélanges de ceux-ci ; et
c) de 15 pour cent à 30 pour cent de plastifiant,
la couche d'enrobage possédant un brillant de surface d'au moins 150.

2. Forme de dosage pharmaceutique comprenant un coeur et une couche d'enrobage à trempage ; ladite couche d'enrobage couvrant sensiblement ledit coeur, **caractérisée en ce que** ladite couche d'enrobage est composée, sur la base du poids sec total de la couche d'enrobage :
a) de 25 pour cent à 80 pour cent de l'agent filmogène hydroxypropylamidon ;
b) de 0,10 pour cent à 33 pour cent d'épaississant choisi dans le groupe constitué de carraghénane kappa, de carraghénane iota, de maltodextrine, de gomme gellane, d'agar-agar, d'amidon gélifiant et de dérivés et de mélanges de ceux-ci ; et
c) de 11 pour cent à 60 pour cent d'un plastifiant,
la couche d'enrobage possédant un brillant de surface d'au moins 150 et ayant une épaisseur de 40 microns à 400 microns.

3. Forme de dosage selon la revendication 2, **caractérisée en ce que** la couche d'enrobage est composée, sur la base du poids sec total de la couche d'enrobage,
a) de 25 pour cent à 80 pour cent d'hydroxypropylamidon ;
b) de 0,1 pour cent à 33 pour cent d'un épaississant choisi dans le groupe constitué de carraghénane kappa, de carraghénane iota, de maltodextrine, de gomme gellane, d'agar-agar, d'amidon gélifiant et de mélanges de ceux-ci ; et
c) de 11 pour cent à 60 pour cent d'un plastifiant choisi dans le groupe constitué de glycérine, de propylèneglycol, de polyéthylèneglycol, de sucres-alcools et de mélanges de ceux-ci.

4. Forme de dosage selon la revendication 1, **caractérisée en ce que** le plastifiant est la glycérine et l'épaississant est le saccharose.

5. Forme de dosage selon la revendication 1, **caractérisée en ce que** la couche d'enrobage est composée, sur la base du poids sec total de la couche d'enrobage,
a) de 40 à 60 pour cent d'un agent filmogène choisi dans le groupe constitué d'amidon de maïs waxy, de dextrine de tapioca, de dérivé d'un amidon de maïs waxy, de dérivé de dextrine de tapioca et de mélanges de ceux-ci ;
b) de 10 pour cent à 20 pour cent de saccharose ; et
c) de 20 pour cent à 25 pour cent de glycérine.

6. Forme de dosage pharmaceutique selon l'une quelconque des revendications précédentes, comprenant en outre une sous-couche couvrant sensiblement ledit coeur, et une couche d'enrobage couvrant sensiblement ladite sous-couche.

7. Forme de dosage selon l'une quelconque des revendications 1 à 6, comprenant une quantité efficace d'un principe actif pharmaceutique, ladite forme de dosage répondant aux exigences de dissolution d'USP pour les formes à libération immédiate dudit principe actif pharmaceutique.

8. Médicament de type capsule simulé comprenant :
a. un coeur ayant une première et une deuxième extrémité,
b. une première couche d'enrobage ayant une première couleur prévue sur ladite première extrémité dudit coeur ;
c. une deuxième couche d'enrobage ayant une deuxième couleur sur ladite deuxième extrémité dudit coeur, ladite première couleur est différente de ladite deuxième couleur ;
**caractérisé en ce qu'**au moins l'une parmi ladite première couche d'enrobage et ladite deuxième couche d'enrobage est la couche d'enrobage telle que décrite dans l'une quelconque des revendications 1 à 5.

9. Méthode de fabrication de comprimés enrobés à partir d'une dispersion aqueuse comprenant la couche d'enrobage telle que décrite dans l'une quelconque des revendications 1 à 5 comprenant le trempage de comprimés dans la dispersion aqueuse.
